Europäisches Patentamt

(19) European Patent Office

Office européen des brevets

(11) Publication number: **0 295 318 B1**

# EUROPEAN PATENT SPECIFICATION

(45) Date of publication of patent specification: **08.05.91**    (51) Int. Cl.⁵: **A61B 7/04**

(21) Application number: **87108732.6**

(22) Date of filing: **17.06.87**

(54) Electronic stethoscopic apparatus.

(43) Date of publication of application:
**21.12.88 Bulletin 88/51**

(45) Publication of the grant of the patent:
**08.05.91 Bulletin 91/19**

(84) Designated Contracting States:
**AT BE CH DE ES FR GB GR IT LI NL SE**

(56) References cited:
**DE-A- 2 607 990**
**DE-A- 3 011 770**
**DE-B- 2 453 926**
**GB-A- 2 124 492**
**US-A- 4 170 717**

(73) Proprietor: **Shue, Ming-Jeng**
**No. 14, Lane 8 Chung-I St.**
**Taichung City(TW)**

(72) Inventor: **Shue, Ming-Jeng**
**No. 14, Lane 8 Chung-I St.**
**Taichung City(TW)**

(74) Representative: **Dreiss, Hosenthien & Fuhlen-
dorf**
**Gerokstrasse 6**
**W-7000 Stuttgart 1(DE)**

## Description

This invention relates to a stethoscopic apparatus designed for multiple auscultation and historical recording of a patient and also designed for clearly transmitting the detected sound from the body portion of a patient.

Stethoscope is an indispensable medical instrument for detecting the sound of a body portion such as heart, lung, trachea, blood vessels, intestine, fetal heart, pulse beat, etc., so as to diagnose the illness of patients by doctors. The structure of the known stethoscope is usually composed of a chest piece with a diaphragm and a bell, a rubber tube connected to the chest piece at one end, and a pair of eartips arranged at another end of the rubber tube through a pair of flexible binaurals. This known stethoscope, though normally used by doctors, has the follwing problems:

1) In order to allow the user to perform the auscultation without interference by external noise, the binaurals of the known stethoscope must be sufficiently resilient, which is uncomfortable for the user. If the resilience of the binaurals is reduced to a certain limit for user's comfort, the relay of the detected sound to the user's ear will be poorer than ever before.

2) In addition to the above-noted defect, the sound detection of the known stethoscope is always made through the chest piece, which is positioned on the patient's body for obtaining the sound and effecting diagnosis. However, as the sound generated from the patient's body is usually very weak and also varies, and transformation of the detected sound is often caused by the known stethoscope, correct diagnosis canot be made easily, and even erroneous diagnosis may be incurred from the transformed sound.

3) Since the known stethoscopes are generally designed for a single user, it is very inconvenient if consultation and/or bed-side teaching is required for the same patient, particularly a female one, because the examination area is confined to a specific spot such as the heart, lung, etc., observers need to perform the auscultation one after another on the specific spot for the same patient. Meanwhile, owing to a different time and different operation of each observer with different types of stethoscopes, various detected sounds may result in various diagnoses. Particularly, when a bed-side teaching has to be conducted, it is difficult to obtain unified diagnosis with the known stethoscopes.

4) Since the known stethoscopes are normally designed to perform auscultation only on the spot, it is hard to make a diagnosis from a very weak and transient sound produced by the body portion of a patient, and, moreover, there is no recording function provided for the known stethoscopes to record the sound required for the patient's medical history for correct diagnosis or for bed-side teaching purpose.

5) It is sometimes necessary to transmit the sound produced by the body portions of a patient to a doctor who is located in a remote place for consultation purpose. However, unfortunately the conventional stethoscopic apparatus do not have proper means to achieve such mechanism.

US-A-4170717 discloses an electronic stethoscope including a transducer coupled via an amplifier circuit to a speaker, which is coupled to earpieces via a flexible duct.

It is a primary object of this invention to provide a multi-functional electronic stethoscopic apparatus with which earphones can be used for performing auscultation without incurring uncomfort to the user's ear.

It is another object of this invention to provide a multi-functional electronic stethoscopic apparatus by which the sound of a patient's viscera can be sensitively detected and amplified for clear transmission to ensure correct diagnosis.

It is a further object of this invention to provide a multi-functional electronic stethoscopic apparatus by which several users can simultaneously perform auscultation for facilitating consultative diagnosis or for teaching purpose.

It is a still further object of this invention to provide a multi-functional electronic stethoscopic apparatus through which the diagnostic condition on the spot, such as the sound of a patient's viscera, the discussions held during consultation or bed-side teaching, etc., can be recorded for detailed study and later reference.

These and other objects of the present invention are achieved by the electronic stethoscope of claims 1 and 4.

Other salient features and advantages of the present invention will become clear from the following detailed description of preferred embodiments with reference to the accompanying drawings, in which:

Figure 1 is a an exploded and perspective view of a first preferred embodiment of an electronic stethoscopic apparatus according to this invention;

Figure 2 is an illustrative view showing the assembly, partly cut-off, of the first preferred embodiment of multi-functional stethoscopic apparatus in Fig. 1.

Figure 3 is a side view of the assembled preferred embodiment of Fig. 1;

Figure 4 is a block diagram illustrating the operational functions of the first preferred embodiment;

Figure 5 is a functional block diagram of the audio wave signal amplifying device and the wireless radio wave transmitting device adopted in the electronic stethoscopic apparatus of the present invention;

Figure 6 is an electric circuit diagram embodying the functional block diagram in Fig. 5 for the audio signal amplifying device and the wireless radio wave transmitting device;

Figure 7 is an exploded and perspective view of a second preferred embodiment of an electronic stethoscopic apparatus according to the present invention; and

Figure 8 is an illustrative view showing the assembly, partly cut-off, of the second preferred embodiment of the stethoscopic apparatus shown in Fig. 2.

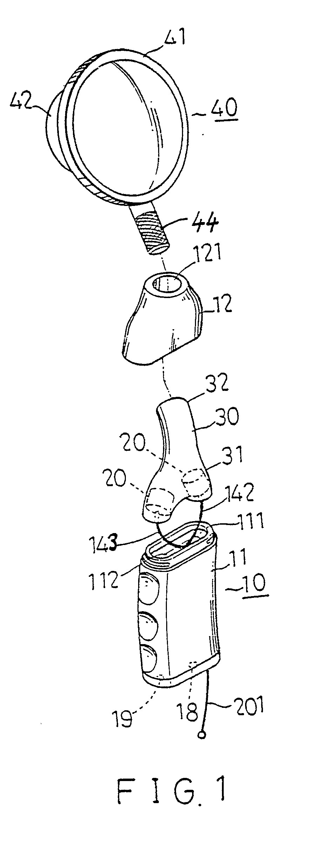

Referring to Figs. 1, 2, 3 and 4, the first preferred embodiment of an electronic stethoscopic apparatus according to this invention comprises: a housing unit 10 composed of a main body 11, an upper cover 12 with a through opening 121, and a lower cover 13; an audio-wave guide 30 provided on top of the main body 11 at one end; and a body contact device 40 fixedly connected to the other end of audio-wave guide.

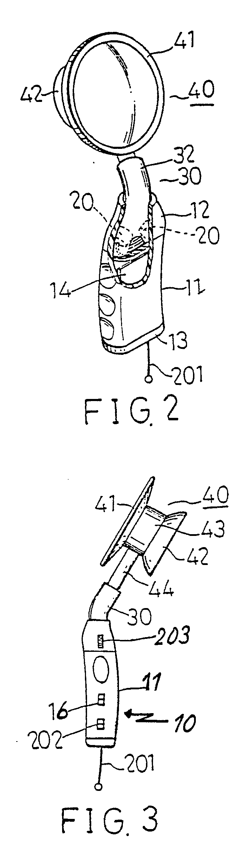

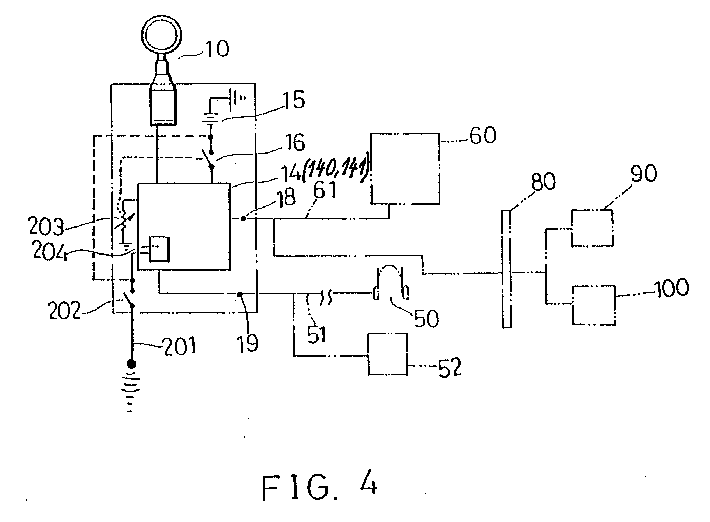

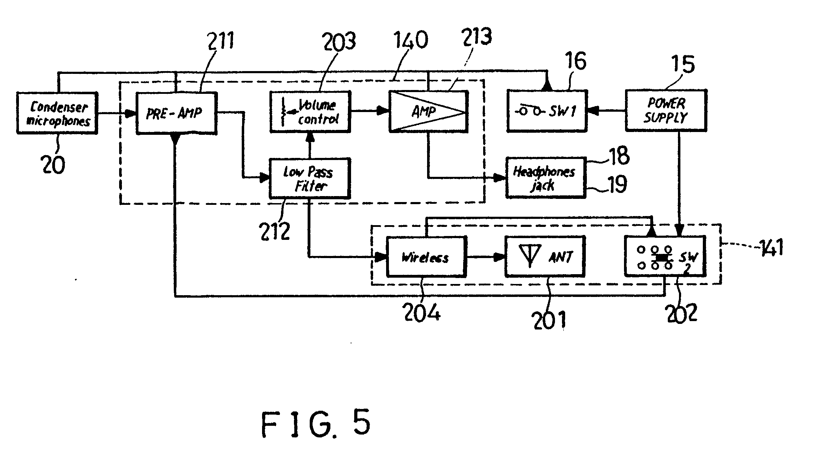

As shown in Figs. 1, 2 and 3 with reference to Figs. 4 and 5, the main body 11, adapted for hand gripping, includes: PC board 14, having audio-signal amplifying device 140 and wireless radio wave transmitting device 141 formed thereon with a plurality of input and output terminals installed in the main body 11, as shown in Fig. 4; a battery supply 15 provided in the main body 11 (batteries can be easily placed and replaced through the lower cover 13) and electrically connected to the audio-signal amplifying device 140 and wireless radio wave transmitting device 141 through power switch l6 and wireless radio on switch 202 respectively installed in a side wall of the main body 11, as shown in Fig. 3; a control knob 203, which is electrically coupled with the audio-signal amplifying device 140, provided at an upper side of the main body 11 for controlling signal output magnitude of the audio-signal amplifying device 140, a recorder jack 18 and an earphone/speaker jack 19 respectively provided in the lower end of the main body 11 and electrically connected to the output terminals of the audio-signal amplifying device 140 as shown in Fig. 1; and a level surface 111 with a central recess 112 formed on top of the main body 11, as shown in Fig. 1.

The audio-wave guide 30, formed in a hollow Y-shape and made of flexible material, includes a lower forked portion 31, having a pair of microphones 20 respectively disposed therein, and an upper branch portion 32 for receiving audio waves. The lower forked portion 31 of the audio-wave guide 30 is properly positioned in the level surface 111 with each output conductive cord 143, 142 of the microphones 20 separately connected to the input terminals of the audio-signal amplifying device 140 through the central recess 112. The upper cover 12 of which the inner section is formed in a shape conforming to that of the audio-wave guide 30, is fixed on the top end of the main body 11 with the audio-wave guide 30 being completely surrounded thereby and being pressed against the level surface 111 of the main body 11 for firm positioning, and with a certain length of the branch portion 32 extending out of the opening 121 of the upper cover 12. It shall be appreciated that the connection between the main body 11 and upper cover 12 as well as the lower cover 13 can be made through a kind of halved joint by which the protrusions provided at both ends of the main body 11 can be easily engaged with the notches respectively formed in an inner wall of the upper cover 12 and the lower cover 13 so that assembly and disassembly of the housing unit 10 can be quickly made therewith.

As shown in Figs. 1 and 3, the body contact device 40, composed of a sound transmission pipe 43 with a diaphragm 41 (for high frequency sounds) on one side, a bell 42 (for low frequency sounds) on the other, and a sound-wave duct 44 in the middle, is sleevingly connected to the upper branch portion 32 through the sound-wave duct, of which the lower end is firmly wrapped by the branch portion 32 of the audio-wave guide 30, and the upper part can be turned in unilateral communication with either the diaphragm 41 or the bell 42.

Referring to Fig. 4 and 5, the wireless radio wave transmitting device 141 includes a wireless radio wave generating circuit 204 formed on the PC board 14, a wireless radio on switch 202 and a wire type antenna 201. The audio wave amplifying device 140 mainly consists of a circuit means which is also formed and arranged on the PC board 14.

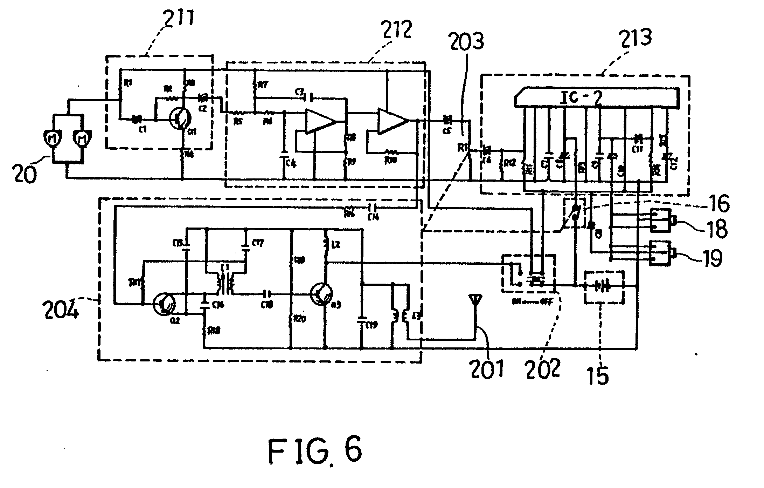

Referring to Fig. 5 and Fig. 6 which show the functional block diagram and the actual circuit diagram of the audio signal amplifying device and the wireless radio wave transmitting device in the stethoscopic apparatus according to the present invention, the audio wave amplifying device 140 comprises a pre-amplifying device 211, a low pass filtering device 212 which is used to filter those signals of frequencies upper than 3000 Hertz, a volume control device 203 which is usually a variable resistor for controlling the output of the signal, and a power amplifying circuit means 213. The wireless radio wave transmitting device 141 comprises a wireless radio wave generating circuit means 204 which is usually an oscillation circuit, an antenna 201 and a wireless radio on switch 202. The filtered electric signal is fed into the wireless radio wave generating circuit 204 for oscillation and transmitted through the antenna 201 when the switch 202 is on. The corresponding electric circuit of the audio wave amplifying device 140 and the wireless radio wave transmitting device 141 is best shown in Fig. 6. Since the circuitry is fallen in the area of the conventional art and also self-explanatory for those skilled in the art to enable the circuit, the operation of this circuitry will not be detailed hereinbelow for the purpose of simplicity.

Operations of the first assembled preferred embodiment of the electronic stethoscopic apparatus according to this invention are as follows:

Referring to Figs. 1,3, 4 and 4, before using the electronic stethoscopic apparatus for auscultation, an earphone 50 for single user or a speaker 52 for group bed-side teaching or consultation can be connected to the audio-signal amplifying device 140 through a signal transmission cord 51 plugged into the earphone/speaker jack 19 of the main body 11. As the situation dictates, the user can select either the diaphragm 41 or the bell 42 along with the audio-wave duct 44 to be placed on the body portion of a patient. Sound detected from the body portion by the body contact device 40 is transmitted through the audio-wave duct 44 and upper branch 32 of the audio-wave guide 30 and received by the microphones 20 from which the sound waves are transduced into electrical signals and fed into the audio-signal amplifying device 140. Upon being amplified by the amplifying device 140, audio signals are transmitted to the earphone 50 for auscultation or to the speaker 52 for consultation and/or bed-side teaching through the earphone/speaker jack 19. In addition, if sound recording is needed for establishing the patient's history or for further study, a recorder 60 can also be connected to the audio-signal amplifying device 140 through another electrical conductive cord 61, which is plugged into the recorder jack 18 of the main body 11. Thus, the amplified audio signals from the audio-signal amplifying device 140 can also be fed into the recorder 60 through the recorder jack 18 for sound recording purpose.

If the detected sound is to be transmitted to a remote place such as for the performance of group bedside teaching of auscultation, the user may turn on the wireless radio on switch 202 and the wireless radio wave transmitting device 141 is then actuated to transmit the signal through the antenna 201. By the wireless radio wave transmitting device 141, remote auscultation and group bedside teaching of auscultation can be conveniently achieved through respectively prepared pocketable receiver and earphone.

It shall be appreciated that since auscultation is made through an earphone, it is much more comfortable for the user than the resilient eartips of the conventional stethoscopes. Further than this, with the arrangement of the highly sensitive microphones 20 and the control knob 203 for controlling the amplitude of the audio-wave volume, the detected sound from the body portion of a patient can be optionally amplified and clearly received by the earphone 50 or the speaker 52 for ensuring the correctness and reliability of diagnosis. Moreover, as the detected sound from the body portion of a patient can be recorded, repeated auscultations can be performed when it is necessary, and medical history can be conveniently established for being used, such as in bed-side teaching, in the future. Furthermore, during bed-side teaching or consultation, simultaneous auscultation can be made through the reproduced sound of the speaker 52 electrically connected to the audio-signal amplifying device 140 through the earphone/speaker jack 19 without requiring repeated individual auscultations as is necessary with the conventional stethoscopes.

In addition to the above-noted features, the preferred embodiment of this invention can also be used to convert the detected sound from a patient's body portion into substantial information so as to effectuate visual diagnosis and establish medical data files. In respect of this arrangement, as shown in Fig. 4, an interface of audio control device 80 is electrically connected to the output of the audio-signal amplifying device 140, and an oscillograph 90 and a plotting instrument 100 can be functionally coupled with the output of the audio control device 80 so that audio signals from the patient's body portion can be converted into visual display information for visual diagnosis and/or data processing operation.

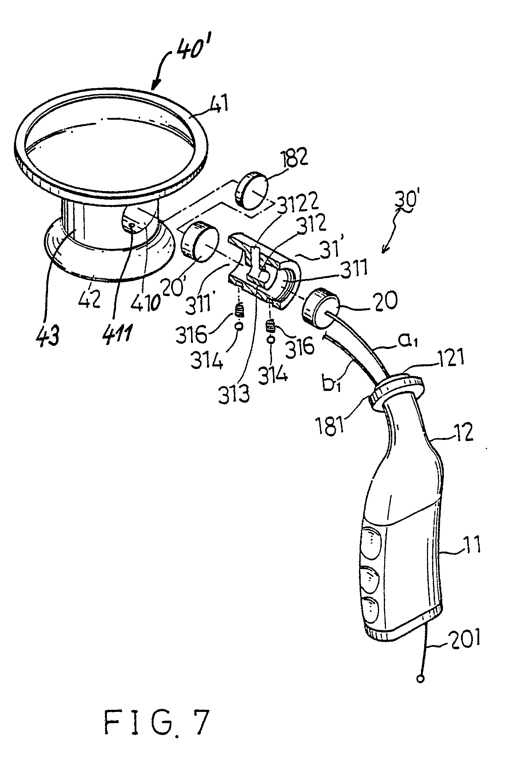

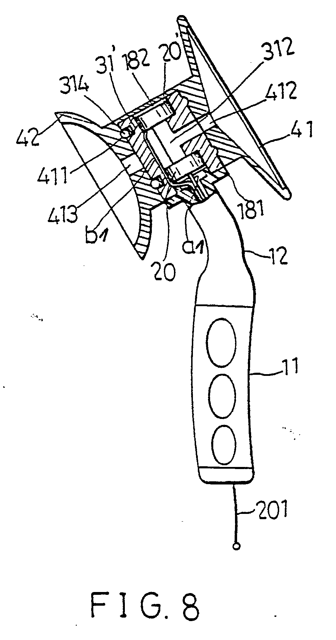

Referring to Figs. 7 and 8 which show the exploded perspective view and partly cut-off sectional view of a second embodiment of the multifunctional electronic stethoscopic apparatus according to the present invention, it is noted that the housing device 11 and most part of the stethoscopic apparatus in the second embodiment are the same in structure as that in the first embodiment stethoscopic apparatus shown in Figs. 1 to 3. The audio wave guide 30' includes a lower cover 181 which is connected to a slightly curved upper portion of the casing 12, a cylinder body 31' and an upper cover 182 which is in connection to the upper chamber 311' of the cylinder body 31'. The cylinder body 31' is properly positioned in the round chamber 410 of the body contact means 40' with the vertical portion 3122 of the three-way opening 312 aligned with the opposing communication opening 412 or 413, and the steel balls 314 biased by springs 316 respectively located in the orifices 411 so as to keep the cylinder body 31' in position in the round chamber 410 of the body contact means 40'. The conductive cords a1 and b1 of the microphones 20, 20', which are separately located in the lower and upper chambers 311, 311', are respectively connected to the audio wave amplifying device through the upper opening 121 of the upper casing 12 with the cord b1 passing through the penetrating hole 313 of the cylinder body 31'. It can be particularly noted that the audio wave guiding means 30' is not like that shown in Figs. 1 to 3 in a Y-shaped configuration. The audio signal amplifying device 140 and the wireless radio wave transmitting device 141 adapted in the first embodiment can be incorporated in the electronic stethoscopic apparatus of the second embodiment shown in Figs. 7 and 8. The operation of the second embodiment electronic stethoscopic apparatus is exactly the same as that of the stethoscopic apparatus shown in the first embodiment.

While a preferred embodiment has been illustrated and described, it will be apparent that many changes may be made in the general construction and arrangement of the invention without departing from the scope of the claims.

## Claims

1. An electronic stethoscopic apparatus having a body contact device (40) with a diaphragm (41) on one side thereof for being placed on a body portion of a patient for auscultation; a housing means (10) adapted for hand gripping and operational control;
   an audio-signal amplifying means (140) having input/output terminals provided therein installed in said housing means (10) for amplifying and outputting audio signals therefrom;
   power supply means (15) replaceably installed in said housing means (10) and electrically connected to said audio-signal amplifying means (140) for providing power supply therefrom;
   and transducing means (20) having output terminals provided thereto, being electrically connected to said audio-signal amplifying means (140) for receiving said sound waves and converting the same into electrical audio signals to be fed into said audio-signal amplifying means (140), characterized by:
   said body contact device (40) having a bell (42) on another side thereof, and a sound-wave duct (44) in a middle portion thereof;
   and an audio-wave guiding means (30) connecting said housing means (10) to the sound-wave duct (44) of the body contact device (40) for receiving and transmitting sound waves therewith to two transducers disposed therein.

2. An electronic stethoscopic apparatus according to Claim 1 wherein said housing means (10) comprises:
   a main body (11) formed in an elongated shape having a level surface (111) with a central recess (112) formed on a top end thereof for positioning said audio-wave guiding means (30) on said level surface (111);
   a lower cover (13) detachably connected to a lower end of the main body (11);
   and an upper cover (12) with its inner section formed in a shape conforming to that of said audio-wave guiding means (30) connected to the top end of the main body (11) for wrappingly pressing a lower portion of said audio-wave guiding means (30) against said level surface (111) with an upper portion of said audio-wave guiding means (30) extending out of the upper cover (12).

3. An electronic stethoscopic apparatus according to Claim 1 or 2 wherein said audio-wave guiding means (30) comprises:
   A Y-shaped body with a forked portion (31) at a lower end thereof for respectively receiving the installation of said transducers (20) therein, and an open branch portion (32) at an upper end for being sleevingly coupled with the body contact means (40) and firmly positioned on said level surface (111) of the main body portion (11) through a pressing force applied by said upper cover (12) after its connection to said main body portion (11); thereby audio waves from the body contact means (40) can be conveniently transferred to said transducers (20).

4. An electronic stethoscopic apparatus having a body contact device (40) with a diaphragm (41) on one side thereof for being placed on a body portion of a patient for auscultation;
   a housing means (10) adapted for hand gripping and operational control;
   an audio-signal amplifying means (140) having input/output terminals provided therein installed in said housing means (10) for amplifying and outputting audio signals therefrom;
   power supply means (15) replaceably installed in said housing means (10) and electrically connected to said audio-signal amplifying means (140) for providing power supply therefrom,
   and transducing means (20) having output terminals provided thereto, being electrically connected to said audio-signal· amplifying means (140) for receiving said sound waves and converting same into electrical audio signals to be fed into said audio-signalamplifying means (140); characterized by:

said body contact means (40') having a bell (42) on another side thereof, and having communicating openings (412,413) provided therein respectively connected to said diaphragm (41) and said bell (42) for sound waves to travel therein;

an audio-wave guiding means (30') rotatably installed in a round chamber (410) separately formed in said body contact device (40') for receiving audio waves from one of the said openings (412, 413) and for passing audio waves therethrough, said transducing means comprising two transducers (20, 20') disposed in said audio-wave guiding means (30');

and an upper cover (12) of said housing means (10) being connected to said audio-wave guiding means (30').

5. An electronic stethoscopic apparatus according to Claim 4, wherein said audio-wave guiding means (30') comprises a lower cover (181) having a central opening (121) formed therein connected to a slightly curved upper portion of said upper cover (12);

a cylinder body (31') positioned in said round chamber (410) of the body contact means (40'); said cylinder body (31') comprising a lower accomodating chamber (311) and an upper accomodating chamber (311') formed respectively at a lower end and an upper end thereof for seperately receiving said transducers (20,20') therein, a three-way communication opening (312) with a vertical portion (3122) aligned with the communicating openings (412) or (413) of the body contact means (40'), a horizontal portion formed in a central portion with two ends aligned with said accomodating chambers (311,311'), and a penetrating hole (313) provided beside the horizontal portion of said three-way communication opening (312) for receiving the conductive cord (b₁) of the transducing means (20') disposed in the upper accomodating chamber (311'); and an upper cover (182) connected to a top end of the upper accomodating chamber (311') of said cylinder body (31') in conjunction with the round chamber (410) of said body contact means (40').

6. An electronic stethoscopic apparatus according to one of the previous claims, wherein a wireless radio wave transmitting device (141) is installed in said housing means (10) for transforming said electrical signal into wireless radio wave signal for being transmitted therefrom.

**Revendications**

1. Appareil stéthoscope électronique comprenant un dispositif de contact avec le corps (40) comportant un diaphragme (41) sur sa face qui doit être posée sur une partie du corps d'un patient à ausculter ; un moyen de boîtier (10) prévu pour être saisi à la main et pour assurer la commande d'utilisation ;

un moyen d'amplification des signaux audio (140) ayant des bornes d'entrée/sortie prévues à l'intérieur dudit moyen de boîtier (10) pour amplifier et délivrer des signaux audio ; un moyen de source d'alimentation (15) susceptible d'être remplacé, installé dans lesdits moyens de boîtier (10) et électriquement connecté audit moyen d'amplification des signaux audio (140) pour alimenter ces derniers ;

et des moyens de transducteur (20) équipés de bornes de sortie, qui sont électriquement connectées audit moyen d'amplification des signaux audio (140) pour recevoir lesdites ondes sonores et les transformer en signaux électriques audio à appliquer audit moyen d'amplification des signaux audio (140), caractérisé en ce que ledit dispositif (40) en contact avec le corps comporte une cloche (42) sur son autre face, et un conduit pour ondes sonores (44) dans sa partie médiane ;

et un moyen de guidage des ondes audio (30) reliant ledit moyen de boîtier (10) et le conduit des ondes sonores (44) du dispositif de contact avec le corps (40) pour recevoir et transmettre avec lui des ondes sonores, lesdits moyens transducteurs étant disposés à l'intérieur.

2. Appareil stéthoscope électronique selon la revendication 1 dans lequel ledit moyen de boîtier (10) comporte :

une partie principale (11) ayant une forme allongée avec une surface lisse (111) dans laquelle est formé un évidement central (112) à l'extrémité supérieure afin de disposer ledit moyen de guidage des ondes audio (30) sur lesdites surfaces lisses (111) ;

un capot inférieur (13) fixé de manière amovible à une extrémité inférieure de la partie principale (11) ;

et un capot supérieur (12) dont la partie intérieure a une forme qui correspond à celle des moyens de guidage des onde audio (30) connectées à l'extrémité supérieure de la partie principale (11) afin d'appuyer en l'enveloppant une partie inférieure desdits moyens de guidage des ondes audio (30) contre ladite surface plane (111) alors qu'une partie supérieure desdits moyens de guidage des ondes audio (30) se prolonge au-delà du capot supérieur (12).

3. Appareil stéthoscope électronique selon l'une des revendications 1 ou 2, dans lequel lesdits moyens de guidage des ondes audio (30) comprennent :

un boîtier en forme de Y avec une partie fourchue (31) à son extrémité inférieure pour recevoir respectivement à l'intérieur lesdits moyens transducteurs (20), et une partie de branche ouverte (32) à une extrémité supérieure qui se trouve couplée par emmanchement dans le moyen de contact avec le corps (40) et solidement fixée sur ladite surface plane (111) de la partie principale (11) par une force de pression appliquée par ledit capot supérieur (12) lorsqu'il a été relié à ladite partie principale (11) ; de façon que les ondes audio provenant du moyen de contact avec le corps (40) puissent être convenablement transférées vers lesdits moyens transducteurs (20).

4. Appareil stéthoscope électronique comportant un dispositif de contact avec le corps (40) ayant un diaphragme (41) sur l'une de ses faces qui doit être posé sur une partie du corps d'un patient à ausculter ; un moyen de boîtier (10) adapté pour être saisi à la main et pour assurer la commande fonctionnelle ;

un moyen d'amplification des signaux audio (140) comportant à l'intérieur des bornes d'entrée/sortie installé dans ledit moyen de boîtier (10) pour amplifier et délivrer des signaux audio ;

un moyen de source d'alimentation remplaçable (15) installé dans ledit moyen de boîtier (10) et électriquement connecté audit moyen d'amplification des signaux audio (140) pour les alimenter,

et des moyens transducteurs (20) équipés de bornes de sortie, qui se trouvent électriquement connectés audit moyen d'amplification des signaux audio (140) pour recevoir lesdites ondes sonores et les transformer en signaux électriques audio qui sont appliqués audit moyen d'amplification des signaux audio (140) ; caractérisé en ce que : ledit moyen de contact avec le corps (40') comporte une cloche (42) sur son autre face, et comporte des ouvertures de communication (412, 413) prévues à l'intérieur respectivement connectées audit diaphragme (41) et à ladite cloche (42) pour permettre le passage des ondes sonores ; un moyen de guidage des ondes audio (30') installé en rotation dans une chambre ronde (410) formée séparément dans ledit dispositif (40') de contact avec le corps pour recevoir des ondes audio provenant de l'une desdites ouvertures (412, 413) et pour se laisser traverser par les ondes audio, ledit moyen transducteur comprenant deux transducteurs (20, 20') placés dans lesdits moyens de guidage des ondes audio (30') ; et un capot supérieur (12) dudit moyen de boîtier (10) étant connecté auxdits moyens de guidage des ondes audio (30').

5. Appareil stéthoscope électronique selon la revendication 4, dans lequel ledit moyen de guidage des ondes audio (30') comporte un boîtier inférieur (181) dans lequel est formée une ouverture centrale (121) connectée à une partie supérieure légèrement cintrée dudit capot supérieur (12) ;

une partie principale cylindrique (31') placée dans ladite chambre ronde (410) dudit moyen de contact avec le corps (40') ; ladite pièce cylindrique (31') comportant une chambre inférieure de logement (311) et une chambre supérieure de logement (311') respectivement formées à son extrémité inférieure et à son extrémité supérieure pour revevoir séparément à l'intérieur lesdits transducteurs (20, 20'), une ouverture de communication à trois directions (312) ayant une partie verticale (3122) alignée avec les ouvertures de communication (412) ou (413) du moyen de contact avec le corps (40'), une partie horizontale formée dans une partie centrale dont les deux extrémités sont alignées avec lesdites chambres de logement (311, 311'), et un orifice de pénétration (313) formé au-dessous de la partie horizontale de ladite ouverture de communication à trois voies (312) pour recevoir le cordon conducteur (b1) dudit moyen transducteur (20') placé dans la chambre supérieure de logement (311') ; et un capot supérieur (182) connecté à une extrémité supérieure de la chambre supérieure de logement (311') de ladite pièce cylindrique (31') en liaison avec la chambre ronde (410) dudit moyen de contact avec le corps (40').

6. Appareil stéthoscope électronique selon l'une des revendications précédentes, dans lequel un dispositif émetteur d'ondes radio sans fil (141) est installé dans ledit moyen de boîtier (10) pour transformer ledit signal électrique en signal d'ondes radio qui sera émis par ce dispositif.

**Ansprüche**

1. Elektronisches Stethoskop, mit einer an ihrer einen Seite eine Membran (41) aufweisenden Körperauflage (40) zum Auflegen auf einen Körperbereich eines Patients zum Auskultieren, mit einem Gehäuse (10), das mit der Hand ergreifbar und mit dem der Betrieb steuerbar ist, mit einer Tonsignal-Verstärkervorrichtung (140), die mit Eingangs/Ausgangsklemmen versehen und im Gehäuse (10) eingebaut ist und zum Verstärken und Abgeben von Tonsignalen dient, mit einer Stromversorgung (15), die im Gehäuse (10) auswechselbar eingebaut und mit der Tonsignal-Verstärkervorrichtung (140) zur Spannungszuführung elektrisch verbunden ist, und mit einer Ausgangsklemmen aufweisenden Transducervorrichtung (20), die mit der Tonsignal-Verstärkervorrichtung (140) elektrisch verbunden ist und zum Empfangen von Schallwellen und zu deren Umwandeln in elektrische Tonsignale, die der Tonsignal-Verstärkervorrichtung (140) zuzuführen sind, dient, **dadurch gekennzeichnet,** daß die Körperauflage (40) an ihrer anderen Seite einen Trichter (42) und in einem mittleren Bereich ein Schallwellenrohr (44) aufweist und daß eine Schallwellen-Leitungsvorrichtung (30) das Gehäuse (10) mit dem Schallwellenrohr (44) der Körperauflage (40) zum Empfangen der Schallwellen und zu deren Übertragen an zwei in ihr enthaltene Transducer verbindet.

2. Elektronisches Stethoskop nach Anspruch 1, dadurch gekennzeichnet, daß das Gehäuse (10) folgendes aufweist:
ein länglich geformtes Hauptteil (11), das eine am oberen Ende gebildete, mit einem mittigen Einschnitt (112) versehene Stirnfläche (111) zum Anbringen der Schallwellen-Leitungsvorrichtung (30) besitzt; eine untere Abdeckung (13), die mit einem unteren Ende des Hauptteils (11) abnehmbar verbunden ist; und eine obere Abdeckung (12), deren innerer Abschnitt eine Form aufweist, die derjenigen der Schallwellen-Leitungsvorrichtung (30), die mit dem oberen Ende des Hauptteils (11) verbunden ist, entspricht, und die dazu dient, daß über einen oberen Bereich der Schallwellen-Leitungsvorrichtung (30), der über die obere Abdeckung (12) hervorsteht, ein unterer Bereich der Schallwellen-Leitungsvorrichtung (30) gegen die Stirnfläche (111) umhüllend gedrückt ist.

3. Elektronisches Stethoskop nach Anspruch 1 oder 2, dadurch gekennzeichnet, daß die Schallwellen-Leitungsvorrichtung (30) folgendes aufweist:
einen Y-förmigen Abschnitt mit einem gabelförmigen Teil (31) an seinem unteren Ende zum Aufnehmen der Transducer (20) und einen offenen Verzweigungsteil (32) an seinem oberen Ende zum überschiebenden Kuppeln mit der Körperauflage (40) und zum festen Anbringen an der Stirnfläche (111) des Hauptkörperteils (11) durch Druckkraft, die auf die obere Abdeckung (12) nach ihrer Verbindung mit dem Hauptkörperteil (11) aufgebracht wird, wodurch Schallwellen von der Körperauflage (40) zu den Transducern (20) in einfacher Weise übertragbar sind.

4. Elektronisches Stethoskop, mit einer an ihrer einen Seite eine Membran (41) aufweisenden Körperauflage (40) zum Auflegen auf einen Körperbereich eines Patients zum Auskultieren, mit einem Gehäuse (10), das mit der Hand ergreifbar und mit dem der Betrieb steuerbar ist, mit einer Tonsignal-Verstärkervorrichtung (140), die mit Eingangs/Ausgangsklemmen versehen und im Gehäuse (10) eingebaut ist und zum Verstärken und Abgeben von Tonsignalen dient, mit einer Stromversorgung (15), die im Gehäuse (10) auswechselbar eingebaut und mit der Tonsignal-Verstärkervorrichtung (140) zur Spannungszuführung elektrisch verbunden ist, und mit einer Ausgangsklemmen aufweisenden Transducervorrichtung (20), die mit der Tonsignal-Verstärkervorrichtung (140) elektrisch verbunden ist und zum Empfangen von Schallwellen und zu deren Umwandeln in elektrische Tonsignale, die der Tonsignal-Verstärkervorrichtung (140) zuzuführen sind, dient, dadurch gekennzeichnet, daß die Körperauflage (40') an ihrer anderen Seite mit einem Trichter (42) versehen ist und Kommunikationsöffnungen (412,413) aufweist, welche mit der Membran (41) bzw. mit dem Trichter (42) verbunden sind und der Ausbreitung der Schallwellen dienen, daß eine Schallwellen-Leitungsvorrichtung (30') in einer runden Kammer (410) drehbar eingebaut ist, welche in der Körperauflage (40') zum Empfangen von Schallwellen aus einer der Öffnungen (412,413) und zum Ausbreiten der Schallwellen für sich gebildet ist, daß die Transducervorrichtung zwei Transducer (20,20') aufweist, die in der Schallwellen-Leitungsvorrichtung (30') angeordnet sind, und daß eine obere Abdeckung (12) des Gehäuses (10) mit der Schallwellen-Leitungsvorrichtung (30') verbunden ist.

5. Elektronisches Stethoskop nach Anspruch 4, dadurch gekennzeichnet, daß die Schallwellen-Leitungsvorrichtung (30') eine untere Abdeckung (181) aufweist, die mit einer zentralen Öffnung (120) versehen ist, welche mit einem leicht gekrümmten oberen Bereich der oberen Abdeckung (12) verbunden ist, daß ein Zylinderkörper (31') in der runden Kammer (410) der Körperauflage (40') angeordnet ist, daß der Zylinderkörper (31') mit einer unteren Aufnahmekammer (311) und einer oberen Aufnahmekammer (311'), die in einem unteren bzw. oberen Ende zur getrennten Aufnahme der Transducer (20,20') gebildet sind, mit einer Dreiwege-Kommunikationsöffnung (312), die einen vertikalen Bereich (3122), der mit den Verbindungsöffnungen (412) oder (413) der Körperauflage (40') fluchtet, und einen horizontalen Bereich, der in einen mittigen Bereich gebildet ist, dessen zwei Enden mit den Aufnahmekammern (311,311') fluchten, besitzt, und mit einer Durchgangsöffnung (313) versehen ist, die neben dem horizontalen Bereich der Dreiwege-Kommunikationsöffnung (312) zum Aufnehmen der Leitungsverbindung (b1) der Transducervorrichtung (20') in der oberen Aufnahmekammer (311') angeordnet ist, und daß eine obere Abdeckung (182) mit dem oberen Ende der oberen Aufnahmekammer (311') des Zylinderkörpers (31') in der runden Kammer (410) der Körperauflage (40') verbunden ist.

6. Elektronisches Stethoskop nach einem der vorhergehenden Ansprüche, dadurch gekennzeichnet, daß eine drahtlose Fernübertragungsvorrichtung (141) zum Umwandeln des elektrischen Signals in ein drahtlos zu übertragendes Funkwellensignal in das Gehäuse (10) eingebaut ist.

41
42
40
44
121
12
32
30
20
31
20
142
111
143
112
11
10
19
18
201

F I G. 1

FIG. 2

FIG. 3

FIG. 4

EP 0 295 318 B1

FIG. 5

FIG. 6

FIG. 7

FIG. 8